# EUROPÄISCHE PATENTSCHRIFT

(11) **EP 0 725 644 B1**
(45) Veröffentlichungstag und Bekanntmachung des Hinweises auf die Patenterteilung: **05.08.1998**
(21) Anmeldenummer: 94928390.7
(22) Anmeldetag: 22.09.1994
(51) Int. Cl.: A61K 31/56

(54) **VERWENDUNG VON BUDESONID ZUR HERSTELLUNG EINES ARZNEIMITTELS ZUR BEHANDLUNG VON ZUSTÄNDEN, DIE AUF EINER VERRINGERTEN ENTERALEN FLÜSSIGKEITSRESORPTION BERUHEN.**
USE OF BUDESONIDE FOR THE PREPARATION OF A MEDICAMENT FOR THE TREATMENT OF CONDITIONS ASSOCIATED WITH REDUCED ENTERAL FLUID RESORPTION
UTILISATION DE BUDESONIDE POUR LA FABRICATION D'UN MEDICAMENT POUR LE TRAITEMENT DES MALADIES ASSOCIEES AVEC LA RESORPTION INTESTINALE DES FLUIDS DIMINUEE

(30) Priorität: 24.11.1993 DE 4340057
(43) Veröffentlichungstag der Anmeldung: 14.08.1996
(73) Patentinhaber: DR. FALK PHARMA GMBH, D-79108 Freiburg (DE)
(72) Erfinder: ECKER, Karl-Wilhelm, D-66450 Bexbach-Kleinottweiler (DE)
(74) Vertreter: Keller, Günter, Dr.
(86) Internationale Anmeldenummer: EP9403165
(87) Internationale Veröffentlichungsnummer: WO9514474

(56) Entgegenhaltungen:
- WO-A-91/16881
- GASTROENTEROL. CLIN. NORTH. AM., Bd.18, Nr.1, 1989 Seiten 21 - 34 D.P. JEWELL 'corticostroids for the management of ulcerative colitis and Crohn's disease.'
- REGUL. PEPTIDE, Bd.49, Nr.2, 1993 Seiten 159 - 166 E. BACCI ET AL. 'Budesonide inhibits plasma extravasation induced by capsaicin and by substance P in the rat nasal mucosa.'
- ALLERGY, Bd.38, Nr.7, 1983 Seiten 461 - 464 M. BENDE ET AL. 'Effect of a topical glucocorticoid, budesonide, on nasal mucosal blood flow as measured with 133Xe wash-out technique.'

## Beschreibung

Die Erfindung betrifft eine neue Indikation des lokalwirksamen Corticosteroids Budesonid (16α 17-Butylidendioxy-1β,21-dihydroxy-1,4-pregnadien-3,20-dion), dessen antientzündliche, antiallergische und antiasthmatische Wirkungen bekannt sind. So wird Budesonid sowohl als lokal-wirksames Antiasthmatikum in Form eines Sprays oder Dosieraerosols als auch zur rektalen Therapie in Form von Einläufen/Klysmen bei Entzündungen des Enddarmes derzeit therapeutisch angewendet. Orale Darreichungsformen zur Behandlung chronisch-entzündlicher Darmerkrankungen befinden sich derzeit in der Erprobungsphase.

Bei Patienten mit Darmresektion besteht sehr häufig das Problem, daß die Flüssigkeitsresorption aus den verbleibenden Darmregionen nicht ausreichend erfolgt. Die Patienten leiden daher nicht nur unter einer dünnflüssigen Ileostoma-Ausscheidung oder einem dünnflüssigen Stuhl und einer erhöhten Stuhlfrequenz, sondern auch ständig unter einer Dehydratation des Körpers mit ungenügender Ionenresorption, so daß die Gefahr einer Exsikkose und komatöser Zustände besteht. Medikamente zur Behandlung bzw. Prävention dieser Zustände sind derzeit nicht bekannt. Es werden lediglich mit unzureichendem Erfolg Medikamente zur Minderung der Darmmotilität (z.B. Loperamid) eingesetzt. Die Wirkung läßt im allgemeinen mit zunehmender Anwendungszeit nach.

Der Erfindung liegt daher die Aufgabe zugrunde, ein Medikament zur Verbesserung der enteralen Flüssigkeitsresorption bereitzustellen. Insbesondere soll die Flüssigkeitsresorption nach postoperativen Zuständen verbessert werden. Das Medikament soll leicht einzunehmen sein und zu einer erheblichen Verbesserung der Lebensqualität der Patienten beitragen. Erfindungsgemäß soll ferner eine funktionssteigernde bzw. funktionsverbessernde Wirkung auf die nicht oder nur geringfügig entzündete Darmschleimhaut ausgeübt werden. Erfindungsgemäß soll das Arzneimittel gut verträglich sein und auch bei längerer Anwendung noch Wirksamkeit zeigen.

Überraschenderweise wurde nun gefunden, daß oral oder rektal verabreichtes Budesonid unabhängig von seiner anti-inflammatorischen Wirkung die Flüssigkeitsresorption im Dünndarm bei Patienten nach Dickdarmresektion wesentlich verbessert. So zeigte sich bei Ileostoma-Patienten, die keinerlei Entzündungsaktivität im Darm aufwiesen, daß die gewöhnlich dünnflüssige Ileostoma-Ausscheidung, die ständig in den Ileostomabeutel abfließt, bereits nach oraler Einnahme von einer bis weniger Budesonid-3mg-Kapseln eine geformte, nicht-flüssige Konsistenz aufwies. Darüber hinaus berichteten diese Patienten über eine wesentlich vermehrte Urin-Ausscheidung. Dies bedeutet, daß die Wasser- bzw. Flüssigkeitsresorption sich unter der Einnahme von Budesonid deutlich verbessert haben muß.

Die Erfindung betrifft somit die Verwendung von Budesonid zur Herstellung eines Arzneimittels zur Behandlung von Zuständen, die auf einer verringerten enteralen Flüssigkeitsresorption beruhen. Insbesondere betrifft sie die Verwendung von Budesonid zur Behandlung von postoperativen Zuständen, die auf einer verringerten enteralen Flüssigkeitsresorption beruhen.

Die gemachte Erfindung, daß Budesonid die enterale Flüssigkeitsresorption verbessert, ist daher von elementarer Bedeutung zur Behandlung und Prävention postoperativer Zustände. Dadurch werden nicht nur ernstliche akute Folgen der Dehydratation des Körpers, sondern auch Nierenschädigungen als Folge einer ständigen Dehydratation verhindert. Darüber hinaus verbessert dies wesentlich die Lebensqualität, das Wohlbefinden und die soziale Komponente des Patienten. Patienten, die hiervon insbesondere profitieren, sind Ileostoma-Patienten, mit und ohne Entzündungsaktivität in den verbleibenden Darmregionen, Patienten mit kontinenter Ileostomie (Kock'-scher Pouch) aber auch Patienten mit einem ileo-analen Pouch, die großteils unter einer vermehrten enteralen Flüssigkeitsausscheidung leiden.

Die gemachte Erfindung an Ileostoma-Patienten läßt den Schluß zu, daß Budesonid einen funktionssteigernden bzw. funktionsverbessernden Effekt auf die nicht oder nur geringfügig entzündete Darmschleimhaut hat. Da auch bei dem häufig verbreiteten "Reizdarmsyndrom" eine latente, klinisch nicht faßbare Entzündungsreaktion vermutet wird, ist auch bei dieser volkswirtschaftlich bedeutenden und äußerst schwierig zu behandelnden gastrointestinalen Funktionsstörung ein "funktionsnormalisierender" Effekt zu erwarten.

Da die Wirkung im wesentlichen in einer Funktionssteigerung oder Funktionsnormalisierung liegt, lassen sich ohne Beschränkung die folgenden weiteren Indikationen aufführen:
1. Diarrhoe nach jeder Art von Darmresektion
   Insbesondere Diarrhoe bei:
   a) distalem Dünndarmresektionssyndrom (Ileocoecalresektion)
   b) Kurzdarmsyndrom
2. Diarrhoe bei Atrophie der Darmschleimhaut
   a) Strahlenenteritis
   b) unter parenteraler Ernährung
   c) unter Zytostatika-Therapie
3. Diarrhoe bei Amyloidose und Morbus Whipple
4. Diarrhoe bei Nebennierenrindeninsuffizienz (Kortikoid-Abhängigkeit der Natriumpumpe)
5. Diarrhoe bei einheimischer und tropischer Sprue
6. Diarrhoe bei HIV-Infektion
7. Diarrhoe infolge exsudativer Enteropathie
   z.B. bei:
   systemischer Mastozytose
   Strahlen-Enteritis (s. auch 2.)
   Zytostase-Therapie (s. auch 2.)
   Purpura Schoenlein-Henoch
   Lupus erythematodes
   Sklerodermie
   Immundefekt-Syndrome (s. auch 6.)
   -Agammaglobulinämie (Hypo-)
      etc.

Budesonid kann zur Behandlung von Zuständen, die auf einer verringerten enteralen Flüssigkeitsresorption beruhen, auf für Budesonid üblichen Wegen verabreicht werden. Bevorzugt wird es oral oder rektal verabreicht. Als orale Verabreichungsformen können beispielsweise erwähnt werden: Kapseln, beispielsweise mit magensaftresistenten Pellets mit verzögerter Wirkstoff-Freisetzung, Tabletten ohne, mit leicht verzögerter oder mit stark verzögerter Freisetzung, magensaftresistentes Granulat mit verzögerter Freisetzung. Als Beispiele für rektale Verabreichungsformen können Klysmen oder Schaum erwähnt werden. Bevorzugt liegt das erfindungsgemäße Arzneimittel in Form von Kapseln mit magensaftresistenten Pellets mit verzögerter Wirkstoff-Freisetzung vor. Die Herstellung dieser Arzneiformen erfolgt nach auf dem Fachgebiet bekannten Verfahren mit pharmazeutisch annehmbaren Hilfsstoffen, z.B. Cellulose-acetatphthalat Eudragit, Maisstärke, Polyvinylpyrrolidon oder Cellulosen, Lactose, Magnesiumstearat, Talk, Kartoffelstärke, Natriumlaurylsulfat oder anderen geeigneten Exzipientien. Weiterhin können die Arzneiformen pharmazeutisch annehmbare Farb- und Aromastoffe sowie Süßstoffe, je nach Bedarf, enthalten.

Eine Dosiseinheit der oralen Darreichungsformen enthält 1 bis 20 mg Budesonid, vorzugsweise 1 bis 12 mg, besonders bevorzugt 3 bis 9 mg Budesonid. Sie wird 1 bis 3 mal täglich, bevorzugt 3 mal täglich verabreicht. Dabei richtet sich die tägliche Dosis nach der Schwere der Erkrankung. Bei schweren Erkrankungen und ungenügender Wirksamkeit bei Dosierungen bis 9 mg pro Tag können bis zu 3 x 6 mg pro Tag verabreicht werden.

Bei rektaler Anwendung kommen Dosiseinheiten von 1 bis 10 mg, bevorzugt 1 bis 3 mg, besonders bevorzugt 2 mg in Betracht. Die rektalen Applikationsformen sollen 1 bis 3 mal täglich, bevorzugt 1 mal täglich abends vor dem Schlafengehen, angewendet werden. Die Tagesdosis und Häufigkeit der Applikation richtet sich nach der Schwere der Erkrankung.

Nachstehend werden einige Beispiele für galenische Formulierungen zur erfindungsgemäßen Verwendung von Budesonid angegeben.

### Beispiel 1

### Tabletten zu 3 mg

| | |
|---|---|
| 1. Budesonid | 3 mg |
| 2. Lactose | 50 mg |
| 3. Maisstärke | 60 mg |
| 4. Polyvinylpyrrolidon | 2 mg |
| | 115 mg |

Die Stoffe 1., 2., 3. werden gut gemischt und mit der Lösung von 4. in 100 mg Ethanol granuliert. Das Granulat wird bei 70°C getrocknet. Aus dem Granulat werden Tabletten zu 115 mg gepreßt.

### Beispiel 2

### Sachets zu 6 mg

| | |
|---|---|
| 1. Budesonid | 6 mg |
| 2. Sorbitol | 280 mg |
| 3. Ascorbylpalmitat | 14 mg |

Die Stoffe 1., 2., 3. werden gut gemischt und mit 30 mg Ethanol granuliert. Das Granulat wird bei 50 bis 60°C getrocknet. Das fertige Granulat wird in Sachets zu 300 mg abgefüllt.

### Beispiel 3

### Kapseln mit gesteuerter Wirkstoff-Freisetzung zu 3 mg

| | |
|---|---|
| 1. Budesonid | 3 mg |
| 2. Zuckerpellets (non paraialles) | 100 mg |
| 3. Polyvinylpyrrolidon | 17 mg |

1. + 3. werden in 100 mg Ethanol gelöst. Die Zuckerpellets werden in einem geeigneten Überzugsgerät vorgelegt und mit der Lösung von 1. + 3. besprüht.

Nach dem Trocknen werden die Wirkstoff-Pellets mit 12,5% Eudragit L in Aceton-Isopropanol (40:60) besprüht. Aufgetragen werden 375 mg Eudragit (L)-Lösung - 30 mg Feststoffanteil. Eudragit L = Polyacrylat (darmlöslich ab pH 6).

Nach dem Trocknen der überzogenen Pellets werden 150 mg Pellets in Hartgelatine-Steckkapseln abgefüllt.

### Beispiel 4

### Budesonid-Klysmen 3 mg/60 ml

| | |
|---|---|
| 1. Budesonid | 3 mg |
| 2. Natriumbenzoat | 300 mg |
| 3. Propylenglykol | 500 mg |
| 4. Methylcellulose | 360 mg |
| 5. Salzsäure q.s. ad pH 6,0 | |
| 6. gereinigtes Wasser ad 60 ml | |

1. wird unter leichtem Erwärmen in 3. gelöst. 2., 4. werden in 6. gelöst. Die beiden Lösungen werden vereinigt und mit 5. auf pH 6 eingestellt. Die fertige Lösung wird in geeignete Klysmenbehälter abgefüllt.

### Beispiel 5

### Budesonid-Schaum 2 mg/5 ml (- 50 ml Schaum)

Eine Dose enthält 10 Hübe zu 2 mg in 5 ml.

| | |
|---|---|
| 1. Budesonid | 20 mg |
| 2. Lanette O | 701 mg |
| 3. Propylenglykol | 3527,0 mg |
| 4. Sorbinsäure | 10 mg |
| | 4258 mg |

1., 2., 4. werden in 3. gelöst (= Wirkstofflösung).
Die Wirkstofflösung wird in Aluminiumblockdosen (Füllvolumen 100 ml) abgefüllt. Dann werden 25 ml eines Propan-Butangemisches (3:5) hinzugegeben. Die Dose wird mit einem geeigneten Delwoventil mit 5 ml Dosierpumpe verschlossen. Mit Stickstoff wird ein Druck von 4,5 bar erzeugt.

## Patentansprüche

1. Verwendung von Budesonid zur Herstellung eines Arzneimittels zur Behandlung von Zuständen, die auf einer verringerten enteralen Flüssigkeitsresorption beruhen.

2. Verwendung nach Anspruch 1 ,dadurch **gekennzeichnet**, daß der Zustand, der auf einer verringerten enteralen Flüssigkeitsresorption beruht, ein postoperativer Zustand ist.

3. Verwendung nach Anspruch 1 oder 2, dadurch **gekennzeichnet**, daß der Zustand eine Diarrhoe ist.

4. Verwendung nach einem der Ansprüche 1 bis 3, dadurch **gekennzeichnet**, daß der Zustand eine Diarrhoe nach jeder Art von Darmresektion, eine Diarrhoe bei Atrophie der Darmschleimhaut, eine Diarrhoe bei Amyloidose und Morbus Whipple, eine Diarrhoe bei Nebennierenrindeninsuffizienz, eine Diarrhoe bei einheimischer und tropischer Sprue, eine Diarrhoe bei HIV-Infektion oder eine Diarrhoe infolge exsudativer Enteropathie ist.

5. Verwendung nach einem der Ansprüche 1 bis 4, dadurch **gekennzeichnet**, daß Budesonid in oral oder rektal verabreichbarer Form verwendet wird.

6. Verwendung nach einem der Ansprüche 1 bis 5, dadurch **gekennzeichnet**, daß Budesonid in Form von Kapseln mit magensaftresistenten Pellets mit verzögerter Wirkstoff-Freisetzung verwendet wird.

7. Verwendung nach Anspruch 6, dadurch **gekennzeichnet**, daß 1 bis 6 mg Budesonid pro Dosiseinheit eingesetzt werden.

## Claims

1. Use of budesonide for the preparation of a medicament for the treatment of conditions associated with a reduced enteral fluid resorption.

2. Use according to claim 1, characterised in that the condition associated with a reduced enteral fluid resorption is a post-operative condition.

3. Use according to claim 1 or 2, characterised in that the condition is a diarrhoea.

4. Use according to one of claims 1 to 3, characterised in that the condition is a diarrhoea after any kind of intestinal resection, a diarrhoea in atrophy of the intestinal mucous membrane, a diarrhoea in amyloidosis and Morbus Whipple, a diarrhoea in insufficiency of the adrenocortical system, a diarrhoea in celiac disease and tropical sprue, a diarrhoea in HIV infection or a diarrhoea as a result of exudative enteropathy.

5. Use according to one of claims 1 to 4, characterised in that budesonide is used in orally or rectally administrable form.

6. Use according to one of claims 1 to 5, characterised in that budesonide is used in the form of capsules containing pellets resistant to gastric juices and having delayed release of the active ingredient.

7. Use according to claim 6, characterised in that 1 to 6 mg budesonide are used per single dose.

## Revendications

1. Utilisation du budésonide pour la fabrication d'un médicament destiné au traitement d'états qui sont à attribuer à une résorption entérale réduite de liquides.

2. Utilisation suivant la revendication 1, caractérisée en ce que l'état qui est à attribuer à une résorption entérale réduite de liquides est un état postopératoire.

3. Utilisation suivant la revendication 1 ou 2, caractérisée en ce que l'état est une diarrhée.

4. Utilisation suivant l'une quelconque des revendications 1 à 3, caractérisée en ce que l'état est une diarrhée après n'importe quelle sorte de résection intestinale, une diarrhée en cas d'atrophie de la muqueuse intestinale, une diarrhée lors d'une amyloïdose et de la maladie de Whipple, une diarrhée en cas d'insuffisance cortico-surrénalienne, une diarrhée en cas d'une sprue indigène et tropicale, une diarrhée lors d'une infection due au VIH ou une diarrhée par suite d'entéropathie exsudative.

5. Utilisation suivant l'une quelconque des revendications 1 à 4, caractérisée en ce que l'on utilise le budésonide sous une forme d'administration par la voie orale ou par la voie rectale.

6. Utilisation suivant l'une quelconque des revendications 1 à 5, caractérisée en ce que l'on utilise le budésonide sous la forme de gélules avec des boulettes résistant au suc gastrique et à libération retardée du principe actif.

7. Utilisation suivant la revendication 6, caractérisée en ce que l'on utilise de 1 à 6 mg de budésonide par unité de dosage.
